# EUROPEAN PATENT SPECIFICATION

(11) **EP 2 322 190 B1**
(45) Date of publication and mention of the grant of the patent: **03.04.2013**
(21) Application number: 09174788.1
(22) Date of filing: 02.11.2009
(51) Int. Cl.: A61K 31/765, A61K 31/80, A61P 1/10

(54) **Compositions for bowel cleansing and use thereof**
Zusammensetzung zur Darmreinigung und deren Verwendung
Agent de nettoyage intestinal et son utilisation

(43) Date of publication of application: 18.05.2011
(73) Proprietor: Promefarm S.r.l., 20129 Milano (IT)
(72) Inventor: Zanarotti, Alessandro, 20129 Milano (IT); Brunetti, Gabriele, 20129 Milano (IT); Cecchetti, Sergio, 20060 Vignate (IT)
(74) Representative: Minoja, Fabrizio

(56) References cited:
- EP-A1- 0 955 065
- WO-A2-2009/052256
- US-A1- 2005 003 021
- E-Z-EM: "LO SO PREP" 30 March 2008 (2008-03-30), XP002559028 Retrieved from the Internet: URL:http://www.ezem.com/pdf/1303811_loso.p df> [retrieved on 2009-12-07]
- TorrinoMedica: "SELG" 28 February 1997 (1997-02-28), XP002559029 Retrieved from the Internet: URL:http://www.torrinomedica.it/studio/gen eraframe.asp?variabile=http://www.torrinom edica.it/farmaci/schedetecniche/SELG.asp> [retrieved on 2009-12-07]
- Wexner SD et al: "A consensus document on bowel preparation prior to colonoscopy" 30 April 2006 (2006-04-30), XP002559030 Retrieved from the Internet: URL:http://www.sages.org/publication/id/BO WEL/> [retrieved on 2009-12-07]

## Description

### Technical field of the invention

The present invention relates to a composition useful for bowel cleansing prior to carry out diagnostic, surgical or therapeutic procedures in the bowel, in particular in the colon (such as for example colonoscopy, radiology and colon surgery).

### Background of the invention

The preparation of bowel, in particular of colon, prior to diagnostic, surgical or therapeutical procedures, for example colonoscopy, is a crucial prerequisite for a successful procedure.

In the past, the bowel preparation was carried out with high volume solutions (7-12 litres) with or without non-absorbable sugars (such as mannitol) which had respectively the inconvenience of causing water retention and promoting the production of potentially dangerous high quantity of gas.

In other cases, hyperosmotic saline laxatives were used, which had a drastic cathartic effect on bowel with the risk, for example, of dehydration, electrolyte imbalances, cardiovascular and renal complications, etc..

Subsequently, a polyethylene glycol (PEG) based solution was first described by the Fordtran's group in '80 (WO87/00754 and Davis GR, Santa Ana CA, Morawski SG, Fordtran JS. Development of a lavage solution associated with minimal water and electrolyte absorption or secretion. Gastroenterology 1980; 78: 991-5).

This formulation has the advantage of being poorly absorbed during gut transit and thus avoiding retention or loss of fluids and electrolytes from the body.

In general, PEGs are compounds of low toxicity and are largely used in pharmaceutical preparations as excipient.

The solution proposed by Fordtran, together with its variants, are a well established preparation for cleansing the colon before diagnostic procedures and they are still the most common preparation used for this purpose.

In order to obtain an adequate bowel preparation, about four litres of this formulation in combination with a diet rich in fluids and poor in fibres are still needed for at least 3 days prior to the procedure.

Furthermore, those formulations are frequently characterized by a poor palatability.

Also a variant of Fordtran's preparation with in addition simethicone has been proposed (Shaver WA, Storms P, Peterson WL. Improvement of oral colonic lavage with supplemental simethicone. Dig Dis Sci 1988;33(2):185-8).

Nevertheless, the quality of the colon preparation is still reported as poor or unacceptable in about 15-20% of the colonoscopies. This is probably due to the fact that 10 to 15% of patients do not complete the preparation because of poor palatability and/or large volume of the administered solution.

Inadequate cleansing of the colon may lead to: a) increased risk of false negative (missed diagnosis), b) more frequent complications, c) longer procedures with increased discomfort for the patient, d) incompleteness of the procedure with the need to repeat the investigation.

There is therefore the need to found new solutions for bowel cleansing with an improved palatability and/or with a reduce total volume to ingest.

EP 1 567 193 - B1 describes a composition comprising, per litre of aqueous solution, from 30 to 350 g polyethylene glycol, from 3 to 20 g of an ascorbic acid component selected from the group consisting of ascorbic acid, a salt of ascorbic acid, or a mixture thereof, an alkali metal or alkaline earth metal sulphate, preferably from 1 to 15 g thereof, and optionally one or more electrolytes selected from sodium chloride, potassium chloride, and sodium hydrogen carbonate, and preferably also comprising flavourings, effective in cleansing the gut in preparation for a endoscopy, especially colonoscopy. Such composition allows the colon cleansing with a reduced volume.

WO-A2009/ 052256 discloses a dry powder composition for reconstitution with water used for bowel cleansing comprising PEG3350, sodium sulphate, sodium chloride, potassium chloride, simethicone as well as aspartame, acesulfam potassium and lemon flavoring; it further comprises ascorbic acid and sodium ascorbate.

The present invention represent a further step toward patient acceptance and compliance.

### Summary of the invention

The present invention relates to a dry composition for reconstitution in water comprising:
(a) polyethylene glycol (PEG),
(b) optionally sodium sulphate,
(c) citric acid,
(d) sodium citrate,
(e) sodium chloride,
(f) potassium chloride,
(g) simethicone.

The invention also concerns the use of a dry composition for reconstitution in water comprising polyethylene glycol (PEG), optionally sodium sulphate, citric acid, sodium citrate, sodium chloride, potassium chloride, simethicone, for the manufacture of a preparation for bowel cleansing.

When the dry composition for reconstitution in water does not comprise sodium sulphate, a laxative is needed to obtain the desired cathartic effect.

Furthermore, it regards an aqueous solution obtainable by reconstitution in water of a dry composition according to the invention.

### Detailed description of the invention

The present invention relates to a dry composition for reconstitution in water comprising:
(a) polyethylene glycol (PEG),
(b) optionally sodium sulphate,
(c) citric acid,
(d) sodium citrate,
(e) sodium chloride,
(f) potassium chloride,
(g) simethicone.

Administration of the reconstituted lavage solution induces watery stools useful for bowel cleansing prior to diagnostic, surgical or therapeutical procedures, in particular to be carried out in the colon, such as for example radiology, colon surgery and in particular colonoscopy.

Citric acid and sodium citrate were included in the formulation for the purpose of improving the taste and thus increasing patient acceptance and compliance. A clinical study was carried out to test the new formulations.

In particular, a clinical study (phase I-II) was performed to investigate the pharmacodynamics and pharmacokinetics of citric acid and sodium citrate in healthy volunteers following administration of a lavage solution according to formulation 1 of present invention. It was found that citric acid and sodium citrate contained in formulation 1 are poorly absorbed as there was no significant increase of urinary excretion of citric acid or citrates. This result is in contrast with the current knowledge on the properties and use of citrates: when administered alone, they are in fact almost entirely absorbed in the small intestine, and are eliminated unchanged in the urine.

When 2 litres of formulation 1 were compared to 4 litres of reference preparation (SELG^{®} 1000), the cathartic effect was found to be equal.

When 2 litres of formulation 1 were compared to 2 litres of reference preparation (SELG^{®} 1000), the cathartic effect of formula was found to be greater than reference preparation.

Thus the study shows an unexpected marked cathartic effect due to the presence of citric acid and sodium citrate (citrates) in the formulation used. In other words, citrates administered as components of the formulation used for the study, are not absorbed and cause a signicant increase of the cathartic effect thus allowing the administration of a low volume solution.

It has been surprisingly found that it is possible to achieve a bowel cleansing satisfactory for carrying out a diagnostic, surgical or therapeutical procedure in the bowel, in particular in the colon, administering a low volume lavage solution comprising polyethylene glycol (PEG), optionally sodium sulphate, citric acid, sodium citrate, sodium chloride, potassium chloride, simethicone.

When the dry composition for reconstitution in water does not comprise sodium sulphate, a laxative is administered few hours, about 3-4 hours, before the administration of the lavage solution.

Therefore, the lavage solution according to the invention is able to induce an equivalent laxative effect at a lower dose with respect to the conventional cleansing solution.

For obtaining a satisfactory bowel, in particular colon, cleansing effect, i.e. the removal of solid residuals and the cleansing of intestinal walls, it is sufficient to administrate the solution according to the invention in a quantity of about 2 litre, whereas 4 litres are to be administered for obtaining a comparable effect when using conventional cleansing solutions.

The presence of citric acid and sodium citrate increases the overall cathartic effect, e.g. increases water stool volume, reducing the total volume of the lavage solution to be taken.

Furthermore, citric acid and sodium citrate improve palatability as well as ease the ingestion of the lavage solution, so that the lavage solution according to the present invention is easier to take and more accepted by patients. Thus, advantageously improving the compliance rate of patients achieving a good and acceptable preparation for the subsequent procedure.

The association with a stimulant laxative, such as for example bisacodyl, reduces the amount of liquid to swallow, i.e. one of the major problems identified by treated patients.

Polyethylene glycol (PEG), also known as Macrogol, has an average molecular weight preferably varying from 3350 to 4000, more preferably the average molecular weight is of 4000.

Preferably, PEG is comprised in a range from 105 g to 121.5 g per litre of aqueous solution.

PEG undergoes virtually no absorption from the gastrointestinal tract and passes unchanged through the intestine. Only very minimal amounts (<0,1%) may be absorbed and are excreted in the urine.

PEG exerts the main osmotic activity, indeed the high molecular weight exerts an osmotic effect retaining water and electrolytes in the intestine.

The unabsorbed fluid accounts for the laxative property by increasing the weight of the fecal effluent.

Also sodium sulphate, citric acid and sodium citrate have osmotic activity. Sodium sulphate anhydrous is preferably used in a range varying from 0 g to 7.5 g per litre, more preferably 7.5 g per litre.

Sodium sulphate is used and useful also for its laxative properties.

Citric acid is a nontoxic substance which is found naturally in the body and is a common ingredient of a normal diet.

The composition may contain anhydrous citric acid from 1.626 to 2.500 g per litre.

Anhydrous sodium citrate may be in the composition in a quantity ranging from 2.132 g to 3.726 g per litre.

The content of sodium chloride is usually from 1.27 g to 1.46 g per litre.

Potassium chloride is normally within a range of from 0.382 g to 0.740 g per litre.

Sodium chloride and potassium chloride are important to prevent absorption or loss of electrolytes and water, maintaining the osmolarity within physiologically acceptable ranges.

As regards simethicone, its therapeutic indications are dyspepsia, relief of flatulence and abdominal discomfort due to excess of gastrointestinal gas and gastroesophageal reflux disease. It is also used as antifoaming agent in radiology or endoscopy of the gastrointestinal tract.

Simethicone is a well established antifoam agent and improves the visualisation of the mucosa.

The laxative may be a stimulant laxative, such as for example bisacodyl, senna, sodium picosulphate.

The laxative may be administered in association with the lavage solution, in such cases the dry composition does not comprise sodium sulphate.

In particular, 10-20 mg of bisacodyl may be administered prior to the intake of about 2 litres of the lavage solution.

The dry composition may normally further comprises proper excipients, such as for example conventional sweeteners, such as acesulfame potassium, etc., and flavours, such as orange, lime, etc., in order to obtain a final product with an acceptable taste.

The dry composition may be in powder, granular or any other suitable form. Powder is the preferred form.

In a preferred embodiment the dry composition may be furnished in unit dosage form, such as for example in a sachet or in a bottle.

The lavage solution of the present invention is mildly hyperosmotic and may have an osmolarity preferably about 400-500 mOsmol/l when the lavage solution comprises sodium sulphate otherwise the osmolarity is about 290 mOsmol/l.

The pH of the lavage solution may be comprised between 3.9 and 5.0, preferably from about 4.3 to 4.8.

In a preferred embodiment, a dry composition reconstituted in water comprising:
(a) polyethylene glycol (PEG),
(b) citric acid,
(c) sodium citrate,
(d) sodium chloride,
(e) potassium chloride,
(f) simethicone,
and a stimulant laxative such as bisacodyl, senna, sodium picosulphate, may be a combined preparation for separate or sequential use in bowel cleansing.

Anyway, when an hyperosmotic solution is administered, it is recommended that additional clear liquid (e.g. water, fruit juice, soft drink, tea, etc.) is taken during the bowel preparation in order to avoid loss of fluid and electrolytes from the body.

It is further provided a possible method for administering the solution containing sodium sulphate (formulation 1) that comprises the following steps:
- If the diagnostic investigation or procedure (for example colonoscopy) is planned early in the morning:
   (a) the day before the exam, at about 15.00 pm, drink 1 L of solution over 1-2 hours (250 ml over 15-20 min.);
   (b) take at least 500 ml of additional clear liquid (water, fruit juice, soft drink, tea/coffee without milk);
   (c) take a rest of about 1-2 hours;
   (d) at about 19.00 pm drink the second litre of solution over 1-2 hours (250 ml over 15-20 min.);
   (e) take at least 500 ml of additional clear liquid.
- If the diagnostic investigation or procedure (for example colonoscopy) is planned late in the morning or in the afternoon:
   (a) the day before the exam, at about 19.00 pm, drink 1 L of solution over 1-2 hours (250 ml over 15-20 min.);
   (b) take at least 500 ml of additional clear liquid (water, fruit juice, soft drink, tea/coffee without milk);
   (c) on the morning of colonoscopy: at about 7.00 or 8.00 am, take 1 L of solution over 1-2 hours (250 ml over 15-20 min.);
   (d) take at least 500 ml of additional clear liquid (water, soft drink).

It is recommended to leave at least two hours between the end of preparation and the time of colonoscopy.

The following non-limitative examples further describe the invention.

### Examples

### 1 Formulation examples

The lavage solution is prepared mixing with water the relevant dry composition up to 500 ml of lavage solution, shaking energically several times to ensure that the ingredients are dissolved and the obtained solution is homogeneous.

The solution is more palatable if chilled before administration. The reconstituted solution should be refrigerated and used within 24-48 hours.

### 1.1 Formulation 1

A dry composition comprising:
52.500 g of PEG 4000,
3.750 g of sodium sulphate anhydrous,
1.863 g of sodium citrate tribasic dihydrate,
0.813 g of citric acid anhydrous,
0.730 g of sodium chloride,
0.370 g of potassium chloride,
0.080 g of simethicone,
0.080 g of lime flavour,
0.130 g of acesulfame potassium.
is reconstituted with water up to 500 ml.

The osmolarity of the lavage solution is about 440 mOsmol/l. 2 litres of the lavage solution are administered together with 1 litre of water or.

### 1.2 Formulation 2

A dry composition comprising:
60.742 g of PEG 4000,
1.066 g of sodium citrate,
1.250 g of citric acid anhydrous,
0.635 g of sodium chloride,
0.191 g of potassium chloride,
0.080 g of simethicone,
0.080 g of silicon dioxide,
0.326 g of orange flavour,
0.129 g of acesulfame potassium.
is reconstituted with water up to 500 ml.

The osmolarity of the lavage solution is about 290 mOsmol/l. 2 litres of the lavage solution are administered after the administration of a stimulant laxative, Bisacodyl.

### 2. Pharmacodynamics

The main active ingredient of the formulation 1 (F1) as well as SELG^{®} 1000, the reference formulation largely clinically employed in Europe, is PEG 4000. The amount of PEG is 105 g vs 58.3 g and of sodium sulphate 7.5 g vs 5.69 g to be dissolved in 1 L of water is higher for F1 than for SELG^{®}; however the total amount of PEG (210 g vs 233.2) and sodium sulphate (15 g vs 22.8 g) to be taken by the patient for the bowel preparation is lower with F1 than with SELG^{®} 1000.

The effect of the formulation 1 at different doses on stool output was determined in phase II study carried out in healthy volunteers.

F1 at the 3 ascending doses T1, T2 and T3 showed a dose-response relationship in terms of total stool weight.

In the efficacy evaluation the highest dose of F1 2 L, was not inferior to the reference product SELG^{®} 1000 in terms of cathartic effect.

The large amount of non-absorbable fluid results in watery diarrhoea, which should efficiently remove any solid residuals from the intestine.

## Claims

1. A dry composition for reconstitution in water comprising:
(a) polyethylene glycol (PEG),
(b) optionally sodium sulphate,
(c) citric acid,
(d) sodium citrate,
(e) sodium chloride,
(f) potassium chloride,
(g) simethicone.

2. A dry composition according to claim 1, wherein the PEG has an average molecular weight ranging from 3350 to 4000.

3. A dry composition according to any one of claims 1 to 2, further comprising a flavour and/or a sweetener.

4. A dry composition according to claim 3, wherein the flavour is orange or lime and the sweetener is acesulfame potassium.

5. A dry composition according to any one of claims 1 to 4, that comprises per 500 ml of aqueous solution:
(a) 52.500 g of PEG 4000,
(b) 3.750 g of sodium sulphate,
(c) 1.863 g of sodium citrate,
(d) 0.813 g of citric acid anhydrous,
(e) 0.730 g of sodium chloride,
(f) 0.370 g of potassium chloride,
(g) 0.080 g of simethicone,
(h) 0.080 g of lime flavour,
(i) 0.130 g of acesulfame potassium.

6. A dry composition according to any one of claims 1 to 5, that comprises per 500 ml of aqueous solution:
(a) 60.742 g of PEG 4000,
(b) 1.066 g of sodium citrate,
(c) 1.250 g of citric acid anhydrous,
(d) 0.635 g of sodium chloride,
(e) 0.191 g of potassium chloride,
(f) 0.080 g of simethicone,
(g) 0.080 g of silicon dioxide,
(h) 0.326 g of orange flavour,
(i) 0.129 g of acesulfame potassium.

7. A dry composition for reconstitution in water comprising:
(a) polyethylene glycol (PEG),
(b) optionally sodium sulphate,
(c) citric acid,
(d) sodium citrate anhydrous,
(e) sodium chloride,
(f) potassium chloride,
(g) simethicone,
for use in bowel cleansing.

8. Use of a dry composition for reconstitution in water comprising:
(a) polyethylene glycol (PEG),
(b) optionally sodium sulphate,
(c) citric acid,
(d) sodium citrate anhydrous,
(e) sodium chloride,
(f) potassium chloride,
(g) simethicone,
for the manufacture of a medicament for bowel cleansing.

9. An aqueous solution obtainable by reconstitution in water of a dry composition of claims 1-6.

10. A dry composition according to any one of claims 1-6 reconstituted in water and a stimulant laxative as a combined preparation for separate or sequential use in bowel cleansing.

11. A combined preparation according to claim 10, wherein the laxative is selected from bisacodyl, senna, sodium picosulphate.

## Patentansprüche

1. Trockene Zusammensetzung für eine Rekonstitution in Wasser, umfassend:
(a) Polyethylenglykol (PEG),
(b) gegebenenfalls Natriumsulfat,
(c) Citronensäure,
(d) Natriumcitrat,
(e) Natriumchlorid,
(f) Kaliumchlorid,
(g) Simethicon.

2. Trockene Zusammensetzung gemäß Anspruch 1, wobei das PEG ein mittleres Molekulargewicht aufweist, das von 3350 bis 4000 reicht.

3. Trockene Zusammensetzung gemäß einem jeglichen der Ansprüche 1 bis 2, die ferner einen Aromastoff und/oder einen Süßstoff umfasst.

4. Trockene Zusammensetzung gemäß Anspruch 3, wobei der Aromastoff Orange oder Limette ist und der Süßstoff Acesulfam-Kalium ist.

5. Trockene Zusammensetzung gemäß einem jeglichen der Ansprüche 1 bis 4, die pro 500 ml an wässriger Lösung:
(a) 52,500 g an PEG 4000,
(b) 3,750 g an Natriumsulfat,
(c) 1,863 g an Natriumcitrat,
(d) 0,813 g an wasserfreier Citronensäure,
(e) 0,730 g an Natriumchlorid,
(f) 0,370 g an Kaliumchlorid,
(g) 0,080 g an Simethicon,
(h) 0,080 g an Limettenaroma,
(i) 0,130 g an Acesulfam-Kalium
umfasst.

6. Trockene Zusammensetzung gemäß einem jeglichen der Ansprüche 1 bis 5, die pro 500 ml an wässriger Lösung:
(a) 60,742 g an PEG 4000,
(b) 1,066 g an Natriumcitrat,
(c) 1,250 g an wasserfreier Citronensäure,
(d) 0,635 g an Natriumchlorid,
(e) 0,191 g an Kaliumchlorid,
(f) 0,080 g an Simethicon,
(g) 0,080 g an Siliziumdioxid,
(h) 0,326 g an Orangenaroma,
(i) 0,129 g an Acesulfam-Kalium
umfasst.

7. Trockene Zusammensetzung für eine Rekonstitution in Wasser, umfassend:
(a) Polyethylenglycol (PEG),
(b) gegebenenfalls Natriumsulfat,
(c) Citronensäure,
(d) wasserfreies Natriumcitrat,
(e) Natriumchlorid,
(f) Kaliumchlorid,
(g) Simethicon,
für eine Verwendung bei einer Darmreinigung.

8. Verwendung einer trockenen Zusammensetzung für eine Rekonstitution in Wasser, umfassend:
(a) Polyethylenglykol (PEG),
(b) gegebenenfalls Natriumsulfat,
(c) Citronensäure,
(d) wasserfreies Natriumcitrat,
(e) Natriumchlorid,
(f) Kaliumchlorid,
(g) Simethicon,
für die Herstellung eines Medikaments zur Darmreinigung.

9. Wässrige Lösung, die durch Rekonstitution in Wasser einer trockenen Zusammensetzung nach den Ansprüchen 1-6 erhältlich ist.

10. Trockene Zusammensetzung gemäß einem jeglichen der Ansprüche 1-6, rekonstituiert in Wasser, und ein stimulierendes Abführmittel als ein Kombinationspräparat für eine separate oder aufeinanderfolgende Verwendung bei einer Darmreinigung.

11. Kombinationspräparat gemäß Anspruch 10, wobei das Abführmittel aus Bisacodyl, Senna, Natriumpicosulfat ausgewählt ist.

## Revendications

1. Composition sèche à reconstituer dans de l'eau, comprenant :
(a) du polyéthylèneglycol (PEG),
(b) éventuellement du sulfate de sodium,
(c) de l'acide citrique,
(d) du citrate de sodium,
(e) du chlorure de sodium,
(f) du chlorure de potassium,
(g) du siméthicone.

2. Composition sèche selon la revendication 1, dans laquelle le PEG a une masse moléculaire moyenne située dans la plage allant de 3350 à 4000.

3. Composition sèche selon l'une quelconque des revendications 1 et 2, comprenant en outre un agent de saveur et/ou un édulcorant.

4. Composition sèche selon la revendication 3, dans laquelle l'agent de saveur est l'orange ou le citron vert et l'édulcorant est l'acésulfame potassium.

5. Composition sèche selon l'une quelconque des revendications 1 à 4, qui comprend, pour 500 ml de solution aqueuse :
(a) 52,500 g de PEG 4000,
(b) 3,750 g de sulfate de sodium,
(c) 1,863 g de citrate de sodium,
(d) 0,813 g d'acide citrique anhydre,
(e) 0,730 g de chlorure de sodium,
(f) 0,370 g de chlorure de potassium,
(g) 0,080 g de siméthicone,
(h) 0,080 g d'agent de saveur citron vert,
(i) 0,130 g d'acésulfame potassium.

6. Composition sèche selon l'une quelconque des revendications 1 à 5, qui comprend, pour 500 ml de solution aqueuse :
(a) 60,742 g de PEG 4000,
(b) 1,066 g de citrate de sodium,
(c) 1,250 g d'acide citrique anhydre,
(d) 0,635 g de chlorure de sodium,
(e) 0,191 g de chlorure de potassium,
(f) 0,080 g de siméthicone,
(g) 0,080 g de dioxyde de silicium,
(h) 0,326 g d'agent de saveur orange,
(i) 0,129 g d'acésulfame potassium.

7. Composition sèche à reconstituer dans de l'eau, comprenant :
(a) du polyéthylèneglycol (PEG),
(b) éventuellement du sulfate de sodium,
(c) de l'acide citrique,
(d) du citrate de sodium anhydre,
(e) du chlorure de sodium,
(f) du chlorure de potassium,
(g) du siméthicone,
pour utilisation dans le nettoyage des intestins.

8. Utilisation d'une composition sèche à reconstituer dans de l'eau comprenant :
(a) du polyéthylèneglycol (PEG),
(b) éventuellement du sulfate de sodium,
(c) de l'acide citrique,
(d) du citrate de sodium anhydre,
(e) du chlorure de sodium,
(f) du chlorure de potassium,
(g) du siméthicone,
pour la fabrication d'un médicament destiné au nettoyage des intestins.

9. Solution aqueuse pouvant être obtenue par reconstitution dans de l'eau d'une composition sèche selon les revendications 1 à 6.

10. Composition sèche selon l'une quelconque des revendications 1 à 6 reconstituée dans de l'eau et laxatif stimulant sous la forme d'une préparation combinée pour utilisation séparée ou successive dans le nettoyage des intestins.

11. Préparation combinée selon la revendication 10, dans laquelle le laxatif est choisi parmi le bisacodyl, le senna, le picosulfate de sodium.
